# EUROPÄISCHE PATENTANMELDUNG

(11) **EP 1 375 490 A1**
(43) Veröffentlichungstag der Anmeldung: **02.01.2004**
(21) Anmeldenummer: 03010256.0
(22) Anmeldetag: 07.05.2003
(51) Int. Cl.: C07D 307/33

(54) **Verfahren zur Herstellung von 2-Acetyl-2-chlor-gamma-butyrolacton**

(30) Priorität: 25.06.2002 DE 10228407
(71) Anmelder: Degussa AG, 40474 Düsseldorf (DE)
(72) Erfinder: Köhler, Günther, Dr., 45770 Marl (DE); Neumann, Manfred, Dr., 45770 Marl (DE)

(57) **Zusammenfassung**

Die Erfindung betrifft ein Verfahren zur Herstellung von 2-Acetyl-2-chlor-γ-butyrolacton ausgehend von 2-Acetyl-γ-butyrolacton in Form seiner Alkali- oder Erdalkalisalze in Gegenwart von Wasser oder Mischungen aus Wasser und Lösemitteln durch Umsetzung mit Chlor.

## Beschreibung

Die Erfindung betrifft ein Verfahren zur Herstellung von 2-Acetyl-2-chlor-γ-butyrolacton (Formel II) durch Umsetzung von einem Alkali- oder Erdalkalisalz (in der Formel formuliert für das Natrium-Salz) des 2-Acetyl-γ-butyrolactons mit Chlor in Gegenwart von Wasser (Formel I)

### Formelschema :

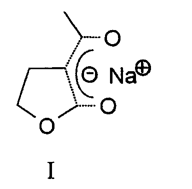

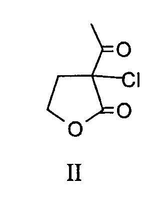

2-Acetyl-2-chlor-y-butyrolacton ist ein wichtiger Rohstoff und Baustein für Pharmawirkstoffe (zum Beispiel Vitamin B1) oder für die Agroindustrie als Herbicid oder Fungicid. Insgesamt ist das Zielprodukt vielseitig zum Aufbau von Heterozyklen geeignet.

So wird die Anwendung von 2-Acetyl-2-chlor-γ-butyrolacton als Edukt in den Patentschriften DE 15 68 177 und US 2 932 653 zur Herstellung von Vitamin B1 sowie in der wissenschaftlichen Literatur von A. Takamizawa et al. in Ann. Rep. Shionogi Res. Lab. 1967, 17, S. 3 - 9 oder von I. N. Rymoreva et al. in Khim -Farm. Zh.16 (9) 1098 - 1102 beschrieben.

Die Herstellung von II wird nach dem Stand der Technik durch Umsetzung von 2-Acetyl-γ-butyrolacton mit Chlor gemäß der Patentschrift DE 15 68 177 (1970) beziehungsweise Ger. Offen. 38 08 024 (1988) oder in Khim. Farm. Zh. 22(2), 231- 234, 1988 beschrieben.

Darüber hinaus wird die Herstellung von II auch durch Umsetzung von 2-Acetyl-γ-butyrolacton mit SOCl₂ im Journal of the Chemical Society, Perkin Transaction 1 (2) 144- 148; 2001 oder in Ger. Offen. 38 08 024 (1988), in J. Med. Chem. 22(3), 306 - 309, 1979 und in Farmaco Ed. Sci. 34(1), 41 - 51, 1979 vorgeschlagen.

Allen diesen Verfahren ist gemeinsam, dass die Selektivität in Bezug auf die Monochlorbildung nicht viel höher als 92 - 95 % ist. Das bei der Reaktion sich bildende dichlorierte 2-Acetyl-γ-butyrolacton als Nebenprodukt bereitet insbesondere in Hinblick auf die Abtrennbarkeit von der monochlorierten Verbindung zur Darstellung eines hochreinen monochlorierten Acetylbutyrolactons große Probleme. Auf Grund der ähnlichen Siedepunktslage sowie der ähnlichen physikalischen Eigenschaften zum monochlorierten Acetylbutyrolacton und vor allem auf Grund der chemischen Reaktivität (zu Polymerisation neigend) des dichlorierten Acetylbutyrolacton ist es nahezu unmöglich, diese beiden Produkte voneinander zu trennen. Für Anwendungen im Bereich der Pharma- aber auch der Agrochemie ist die wirtschaftlich günstige Herstellung einer hochreinen Monochlorverbindung des 2-Acetyl-γ-butyrolactons jedoch erstrebenswert.

Es bestand daher die Aufgabe, eine Synthese mit hoher Selektivität in Bezug auf die monochlorierte Verbindung zu entwickeln.

Überraschend gelingt die Lösung der Aufgabe dadurch, dass die Salze des 2-Acetyl-γ-butyrolactons von Alkali- oder Erdalkalimetallen als Edukte für die Herstellung von 2-Acetyl-2-chlor-γ-butyrolacton in Wasser allein oder in einer Mischung aus Wasser und protischen oder aprotischen Lösemitteln mit gasförmigem oder flüssigem Chlor umgesetzt werden. Vorzugsweise können mit Wasser mischbare Lösemittel verwendet werden, zum Beispiel niedermolekulare Alkohole wie Methanol, Ethanol, n- und iso-Propanol, Ether wie Tetrahydrofuran oder p-Dioxan oder Ester wie Essigsäuremethylester. Bei der Umsetzung wird dabei ein sehr reines 2-Acetyl-2-chlor-γ-butyrolacton mit einer Selektivität von >98 % erhalten. Als Nebenprodukt fällt eine wässrige oder mit anderen Lösemitteln entsprechende Lösung/Mischung der Alkali- oder Erdalkalichloride an, die als Lösung mit Wasser als Hauptbestandteil einfach entsorgt werden können.

Gegenstand der Erfindung ist also ein Verfahren zur Herstellung von 2-Acetyl-2-chlor-γ-butyrolacton ausgehend von 2-Acetyl-γ-butyrolacton in Form seiner Alkali- oder Erdalkalisalze in Gegenwart von Wasser oder Mischungen aus Wasser mit protischen oder aprotischen Lösemitteln durch Umsetzung mit Chlor.

Vorteilhaft ist dieses Verfahren zusätzlich dadurch, dass alle technischen und kommerziellen Verfahren zur Herstellung von 2-Acetyl-γ-butyrolacton gewöhnlich über die Bildung der Alkalisalze verlaufen, wobei es zur Protonierung des freien 2-Acetyl-γ-butyrolactons durch Behandlung beziehungsweise Neutralisation mit Säuren unter Bildung eines Äquivalentes Salzes kommt (DE 196 06 975).
Anschließend wird zur Herstellung von 2-Acetyl-2-chlor-γ-butyrolacton durch Umsetzung von 2-Acetyl-γ-butyrolacton mit Chlor nach dem Stand der Technik ein stöchiometrisches Äquivalent Chlorwasserstoff frei, das in technischen Synthesen gewöhnlich mit einer Base wie zum Beispiel Natronlauge neutralisiert und als wässrige Lösung von Natriumchlorid oder als festes Nebenprodukt entsorgt wird. Damit wird grundsätzlich ein weiteres Äquivalent Salz über beide Synthesestufen erzeugt.

Ein weiterer Vorteil des hier geschilderten Verfahrens ist demnach die Bildung nur eines Äquivalentes Salzes über beide Synthesestufen, wodurch die Kopplung beider Verfahren gemäß vorliegender Erfindung zusätzlich kostengünstiger und umweltschonender ist.

Der Wassergehalt beträgt 0,1 bis 99 Gew.-%, vorzugsweise 5 bis 95 Gew.-% (bezogen auf die Reaktionsmischung).

Das Chlor kann flüssig oder gasförmig eingesetzt werden.

Die Reaktionstemperatur beträgt 0 -100 °C, vorzugsweise 10 - 80 °C.

Die Reaktion kann im Druckbereich von 0,5 bis 5 bar durchgeführt werden. Vorteilhaft wird jedoch bei Normaldruck gearbeitet.

Der Anteil des Salzes im Wasser oder in den Mischungen aus Wasser mit einem oder mehreren und und dem Alkali- beziehungsweise Erdalkalisalz vom 2-Acetyl-γ-butyrolacton beträgt 0,1 bis 50 Gew.-%, vorzugsweise 1 bis 40 Gew.-%.

Als Alkalimetalle können Li, Na und K und als Erdalkalimetalle Mg oder Ca als Kationen mit dem 2-Acetyl-γ-butyrolacton vorliegen.

Das Verfahren kann absatzweise oder kontinuierlich betrieben werden.

Die Reaktions- beziehungsweise Verweilzeit kann 5 Minuten bis 20 Stunden, vorzugsweise 30 Minuten bis 6 Stunden, betragen.

### Beispiel:

150 g (1 mol) des Natriumsalzes vom 2-Acetyl-γ-butyrolacton wurden in 600 g Wasser gelöst und bei 20 - 30 °C mit 77 g (1,1 mol) gasförmigem Chlor innerhalb von 3 Stunden behandelt. Nach Abschluss der Chloreinleitung in diese Mischung wurden bei Raumtemperatur die zwei flüssigen Phasen getrennt, wobei die organische Phase destillativ aufgearbeitet wurde. Die wässrige Phase enthielt NaCl, das als Kochsalzlösung entsorgt werden konnte.

Aus der destillativen Aufarbeitung der organischen Phase (Destillationsapparatur bestehend aus einen 500 ml Kolben und einer 10 cm Kolonne mit einer Raschigringfüllung und Rücklaufteiler) wurden im Vorlauf beziehungsweise als erste Fraktion wenig Wasser und ca. 7,5 g unumgesetztes 2-Acetyl-γ-butyrolacton sowie im Hauptlauf der Destillation 153 g 2-Acetyl-2-chlor-γ-butyrolacton mit einer Reinheit von 98,9 % erhalten. Als Verunreinigung waren ca. 0,2 % 2-Acetyl-γ-butyrolacton und 0,9 % dichloriertes 2-Acetyl-γ-butyrolacton enthalten.

## Patentansprüche

1. Verfahren zur Herstellung von 2-Acetyl-2-chlor-γ-butyrolacton,
**dadurch gekennzeichnet,**
**dass** 2-Acetyl-γ-butyrolacton in Form seiner Alkali- oder Erdalkalisalze in Gegenwart von Wasser oder Mischungen aus Wasser mit Lösemitteln mit Chlor umgesetzt wird.

2. Verfahren nach Anspruch 1,
**dadurch gekennzeichnet,**
**dass** Chlor flüssig oder gasförmig angewendet wird.

3. Verfahren nach einem der vorhergehenden Ansprüche,
**dadurch gekennzeichnet,**
**dass** im Wasser oder in den Mischungen aus Wasser mit Lösemitteln und dem Alkalibeziehungsweise Erdalkalisalz vom 2-Acetyl-γ-butyrolacton das Salz mit einem Anteil von 0,1 % bis 50 % enthalten ist.

4. Verfahren nach einem der vorhergehenden Ansprüche
**dadurch gekennzeichnet,**
**dass** als Alkalimetalle Li, Na und K und als Erdalkalimetalle Mg oder Ca als Kationen mit dem 2-Acetyl-γ-butyrolacton vorliegen.

5. Verfahren nach einem der vorhergehenden Ansprüche,
**dadurch gekennzeichnet,**
**dass** der Wassergehalt 0,1 bis 99 Gew.-%, vorzugsweise 5 bis 95 Gew.-% (bezogen auf die Reaktionsmischung) beträgt.

6. Verfahren nach Anspruch 1,
**dadurch gekennzeichnet,**
**dass** die Reaktionstemperatur 0 - 100 °C beträgt.

7. Verfahren nach einem der vorhergehenden Ansprüche,
**dadurch gekennzeichnet,**
**dass** die Reaktions- beziehungsweise Verweilzeit 5 Minuten bis 20 Stunden beträgt.

8. Verfahren nach Anspruch 1,
**dadurch gekennzeichnet,**
**dass** das Verfahren absatzweise oder kontinuierlich betrieben wird.
